# EUROPÄISCHE PATENTSCHRIFT

(11) **EP 2 772 478 B1**
(45) Veröffentlichungstag und Bekanntmachung des Hinweises auf die Patenterteilung: **13.04.2016**
(21) Anmeldenummer: 14154590.5
(22) Anmeldetag: 11.02.2014
(51) Int. Cl.: B01J 23/38, C07C 45/58, C07C 49/413, B01J 21/06, B01J 23/42, B01J 23/44, B01J 23/46, C07B 41/06, B01J 23/40

(54) **Verfahren zur Herstellung von Ketonen aus Epoxiden**
Process for preparing ketones from epoxides
Procédé de fabrication de cétones à partir d'époxydes

(30) Priorität: 01.03.2013 DE 102013203470
(43) Veröffentlichungstag der Anmeldung: 03.09.2014
(73) Patentinhaber: Evonik Degussa GmbH, 45128 Essen (DE)
(72) Erfinder: Micoine, Kévin, Dr., 45739 Oer-Erkenschwick (DE); Roos, Martin, Dr., 45721 Haltern am See (DE); Hannen, Peter, Dr., 45701 Herten (DE); Häger, Harald, Dr., 59348 Lüdinghausen (DE); Bartosch, Klaus, 45721 Haltern am See (DE)

(56) Entgegenhaltungen:
- EP-A1- 1 090 900
- EP-A2- 1 018 498
- WO-A1-2012/166335
- WO-A1-2013/013704
- US-A1- 2002 107 142
- WEIZHEN LI ET AL: "Structures and Properties of Zirconia-Supported Ruthenium Oxide Catalysts for the Selective Oxidation of Methanol to Methyl Formate", THE JOURNAL OF PHYSICAL CHEMISTRY B, Bd. 110, Nr. 46, 1. November 2006 (2006-11-01), Seiten 23337-23342, XP055123218, ISSN: 1520-6106, DOI: 10.1021/jp0648689
- BITTER J H ET AL: "The State of Zirconia Supported Platinum Catalysts for CO2/CH4Reforming", JOURNAL OF CATALYSIS, ACADEMIC PRESS, DULUTH, MN, US, Bd. 171, Nr. 1, 1. Oktober 1997 (1997-10-01), Seiten 279-286, XP004468804, ISSN: 0021-9517, DOI: 10.1006/JCAT.1997.1792
- TIWARI V ET AL: "One-step synthesis of noble metal-titanium dioxide nanocomposites in a flame aerosol reactor", APPLIED CATALYSIS A: GENERAL, ELSEVIER SCIENCE, AMSTERDAM, NL, Bd. 345, Nr. 2, 1. August 2008 (2008-08-01), Seiten 241-246, XP022808078, ISSN: 0926-860X, DOI: 10.1016/J.APCATA.2008.05.003 [gefunden am 2008-05-14]

## Beschreibung

Die vorliegende Erfindung betrifft ein Verfahren zur Herstellung eines Ketons aus einer mindestens eine Epoxidgruppe enthaltenden aliphatischen oder cycloaliphatischen Verbindung E sowie ein Verfahren zur Synthese von Lactamen.

Die Öffnung von Oxiranringen mit saueren bzw. basischen Oxiden ist bekannt (V.S. Joshi, N.P. Damodaran, S. Dev, Tetrahedron 1971, 27, 459-474; K. Arata, K. Tanabe, Bull. Chem. Soc. Jpn. 1980, 53, 299-303; K. Arata, H. Nakamura, Y. Nakamura, Bull. Chem. Soc. Jpn. 1994, 67, 2351-2353). Aus einer Epoxidgruppen enthaltenden Verbindung wie Monoepoxy-cyclododecan (CDAN-Epoxid) wird zunächst der Allylalkohol (3-Cyclododecenol; CDENOL) erhalten. Anschließend erfolgt die Umsetzung zum Keton (Cyclododecanon; CDON).

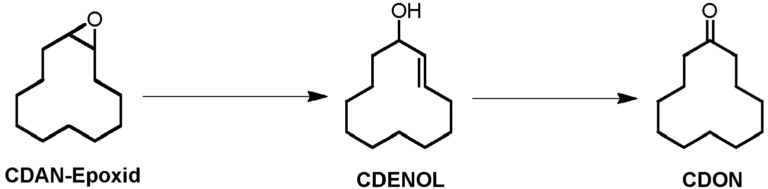

Bei dieser zweistufigen Reaktion tritt das Problem auf, dass neben dem cyclischen Allylalkohol dehydratisierte Diene oder Dimere als Nebenprodukte entstehen.

Eine selektive Umsetzung des Epoxids zum Keton wurde von Chernyshkove und Mushenko berichtet (Chernyshkove, F.A.; Mushenko, D.V. Neftekhimiya 1976, 16, 250-4). Hierzu wurden die Epoxide in einer Wasserstoffatmosphäre über Palladium- und Rhodium-Katalysatoren, die auf Aluminiumoxid oder Kohle geträgert waren, zur Reaktion gebracht. Die Ketone wurden bei vollständigem Umsatz des Edukts mit Ausbeuten von über 80 % erhalten.

In EP-A-1090900 wird die Reaktion von CDAN-Epoxid zu einem Gemisch aus CDON und Cyclododecanol (CDOL) beschrieben. Hierzu werden Platingruppenmetalle, ein inerter Träger und ein Promoter eingesetzt. Die Reaktionen verlaufen in einer Wasserstoffatmosphäre.

EP-A-1018498 offenbart die Umsetzung cyclischer Epoxide mit Wasserstoff und Platingruppenmetallen in einer Wasserstoffatmosphäre. Hierdurch wird ebenfalls ein Keton-/Alkohol-Gemisch gewonnen. Der Druck des Wasserstoffs beträgt mindestens 1 bar. Die Schrift lehrt, dass geringere Drücke eine zu lange Reaktionszeit erfordern würden.

Es bestand nunmehr die Aufgabe, die Gewinnung von Ketonen aus Epoxiden derart anzupassen, dass ein hoher Anteil an Keton bei geringem Alkohol-Anteil erhalten wird. Weiterhin sollten Nebenprodukte, insbesondere ungesättigte Nebenprodukte, in möglichst geringer Konzentration entstehen. Zudem sollte die Reaktion im Vergleich zum Stand der Technik technisch weniger aufwändig sein. Hierbei sollte die zweistufige Reaktion zum Keton ohne Isolierung des Allylalkohols erfolgen können. Die Reaktionszeiten sollten den Zeiten des Standes der Technik entsprechen.

Demgemäß wurde ein Verfahren zur Herstellung eines Ketons aus einer mindestens eine Epoxidgruppe enthaltenden Verbindung E unter Einsatz eines Gemischs umfassend mindestens ein Edelmetall und mindestens ein Metalloxid als Katalysatorsystem gefunden, wobei das Metalloxid Titandioxid, Zirconiumdioxid oder Mischungen der beiden Oxide enthält oder daraus besteht. Zirconiumdioxid ist bevorzugtes Metalloxid.

Das erfindungsgemäße Verfahren stellt eine heterogene Katalyse dar.

Die Verbindung E kann aliphatisch oder cycloaliphatisch sein, wobei cycloaliphatische Verbindungen bevorzugt sind. Vorzugsweise sind 4 bis 20 C-Atome, bevorzugt 6 bis 16 C-Atome, besonders bevorzugt 8 bis 14 C-Atome, ganz besonders bevorzugt 10 bis 12 C-Atome und insbesondere 12 C-Atome umfasst.

Die Verbindung E kann eine oder mehrere Epoxid-Gruppen enthalten, wobei Mono-Epoxid-Verbindungen bevorzugt sind.

Weiterhin kann die Verbindung gesättigt oder ungesättigt sein. Beispielsweise können eine oder zwei Doppelbindungen enthalten sein.

Bevorzugte Verbindungen E sind Monoepoxy-Cycloalkane, Monoexpoxy-Cycloalkandiene und Monoepoxy-Cycloalkene, wobei MonoepoxyCycloalkane besonders bevorzugt sind. Eine ganz besonders bevorzugte Verbindung E ist Monoepoxy-cyclododecan (CDAN-Epoxid).

Es hat sich gezeigt, dass die Bildung des entsprechenden Alkohols als Nebenprodukt vom Druck des Wasserstoffs abhängt: Mit steigendem Druck erhöht sich der Alkohol-Anteil, so dass die Keton-Selektivität abnimmt.

Das erfindungsgemäße Verfahren wird bei einem Wasserstoffdruck von 0 bis 0,9 bar, ganz besonders bevorzugt 0 bis 0,5 bar, durchgeführt. Das erfindungsgemäße Verfahren kann ohne Wasserstoff durchgeführt werden, es ist jedoch zur Unterbindung ungesättigter Nebenprodukte bevorzugt, zumindest einen geringen Wasserstoffanteil vorzulegen. Dieser kann 0,05 bis 0,5 bar, vorzugsweise 0,1 bis 0,4 bar betragen.

Ein Verfahren ohne Wasserstoff bzw. mit einem Wasserstoffdruck von maximal 0,9 bar ist insbesondere für Katalysatorsysteme umfassend Titandioxid vorteilhaft.

Die zuvor genannten Druckangaben beziehen sich auf den Partialdruck an Wasserstoff in dem System. Üblicherweise sind Komponenten des Reaktionsgemisches, einschließlich des Lösemittels, Luft oder Inertgase wie Stickstoff oder Argon weitere gasförmige Bestandteile des Systems.

Durch die niedrigen Wasserstoffdrücke ist gegenüber dem Stand der Technik ein deutlich geringerer technischer Aufwand insbesondere bei der geeigneten Apparatur notwendig, um mit Wasserstoff arbeiten zu können. Der besondere Vorteil der Erfindung liegt darin, dass das Keton in hohen Ausbeuten ohne die Anwesenheit von Wasserstoff gewonnen werden kann.

Die Temperatur während der Reaktion wird vorzugsweise auf einen Bereich von 100 bis 350 °C, bevorzugt 175 bis 275 °C und besonders bevorzugt zwischen 200 und 250°C eingestellt. Die Reaktion kann durchgeführt werden mit einer Verbindung E, die sich im flüssigen oder gasförmigen Zustand befindet.

Das Edelmetall des Katalysatorsystems wird vorzugsweise ausgewählt aus Ruthenium, Rhodium, Palladium, Osmium, Iridium und Platin, wobei Ruthenium, Palladium und Platin bevorzugt sind und Palladium besonders bevorzugt ist. Das Edelmetall kann als Pulver (ungeträgert) oder geträgert vorliegen. In Pulverform sind beispielsweise elementare Edelmetalle oder ihre Oxide geeignet.

Weiterhin ist mindestens ein Metalloxid als weiterer Bestandteil des Katalysatorsystems enthalten. Das Metalloxid des Katalysatorsystems umfasst Titandioxid, Zirconiumdioxid oder Mischungen daraus oder besteht aus mindestens einem der zuvor genannten Oxide. Hiervon sind auch mit Titandioxid oder Zirconiumdioxid dotierte oder beschichtete Stoffe wie Aluminiumoxid oder Siliciumdioxid umfasst.

Das Metalloxid des Katalysatorsystems kann als Träger für das Edelmetall des Katalysatorsystems fungieren. Das Edelmetall kann wahlweise auf einem alternativen Träger aufgebracht sein, der beispielsweise aus Aluminiumoxid, Siliciumdioxid oder Aktivkohle ausgewählt ist. Titandioxid oder Zirconiumdioxid sind bevorzugte Träger.

Die Metalloxide des Katalysatorsystems sowie die alternativen Träger können als Pulver oder als Formkörper vorliegen. Geeignete Formkörper sind Kugeln, Extrudate, Tabletten, Granulate und Pellets. Es ist bevorzugt, dass die Träger des Edelmetalls als Formkörper vorliegen. Es ist ebenso bevorzugt, dass das Metalloxid des Katalysatorsystems, wenn es nicht als Träger fungiert, als Formkörper vorliegt.

Unabhängig davon, ob das Edelmetall geträgert ist oder nicht und welcher Träger zum Einsatz kommt, ist es für die Erfindung wesentlich, dass zumindest ein Metalloxid des Katalysatorsystems enthalten ist.

Das Katalysatorsystem kann folglich unabhängig voneinander als eines der folgenden Systemformen vorliegen:
I) Das Edelmetall ist nicht geträgert; als Metalloxid des Katalysatorsystems ist mindestens Titandioxid oder Zirconiumdioxid enthalten;
II) das Edelmetall ist geträgert, wobei der Träger nicht Titandioxid und/oder Zirconiumdioxid enthält oder daraus besteht. Das System enthält zusätzlich zumindest ein Metalloxid, welches ausgewählt ist aus Titandioxid oder Zirconiumdioxid.
III) Das Edelmetall ist auf einem Metalloxid geträgert, welches aus Titandioxid und Zirconiumdioxid ausgewählt ist.

Die Systemformen II und III sind bevorzugt, wobei Systemform III besonders bevorzugt ist.

Geeignetes Titandioxid als Metalloxid des Katalysatorsystems kann durch das Sulfatverfahren, das Chloridverfahren oder durch Flammhydrolyse (Pyrogenverfahren) von Titantetrachlorid erhalten werden. Sämtliche Verfahren sind dem Fachmann bekannt. Geeignete Modifikationen sind Rutil und Anatas, wobei das eingesetzte Titandioxid Mischungen der genannten Modifikationen enthalten kann.

Das durch das Sulfat- oder Chloridverfahren hergestellte Titandioxid kann in Wasser sauer reagieren, wobei die Verbindungen üblicherweise einen pH-Wert von 3 oder weniger aufweisen (saures Titandioxid). Ebenso enthält saures Titandioxid meist mehr als 5 Gew.-%, bezogen auf das Gesamtgewicht des Titandioxid-Trägers, an Stoffen wie Titanylsulfat oder Titanylhydroxid auf. Ein Titandioxid auf Basis eines sauren Titandioxids ist kommerziell als Aerolyst 7750 erhältlich (Evonik, Deutschland). Saures Titanoxid ist für das vorliegende Verfahren weniger bevorzugt. In anderen Worten ist es bevorzugt, kein saures Titandioxid einzusetzen. Geeignetes, nicht-saures Titandioxid, welche bevorzugt ist, zeigt in Wasser einen pH-Wert von 5 oder mehr.

Besonders bevorzugtes Titandioxid wird mittels Flammpyrolyse gewonnen, wie es beispielsweise in DE-A-830786 beschrieben ist.

Geeignetes Titandioxid ist unter der Bezeichnung Aeroxid P25 Titandioxid (Pulver) oder Aerolyst 7711 (Formkörper) der Firma Evonik, Deutschland, sowie Hombikat M234 (Formkörper) der Firma Sachtleben, Deutschland, erhältlich.

Zirconiumdioxid (Zirconium(IV)-oxid) ist beispielsweise aus Zirconiumhydroxid erhältlich, wobei es bei über 200 °C, beispielsweise bei 350 °C, kalziniert wird. Das Zirconiumdioxid kann beispielsweise mit Yttriumoxid dotiert sein.

Geeignetes Zirconiumdioxid ist monoklin oder tetragonal. Mischungen dieser Modifikationen sind möglich.

Das Metalloxid des Katalysatorsystems kann eine durchschnittliche Schüttdichte von 0,5 bis 2 g/cm³ aufweisen.

Das Metalloxid des Katalysatorsystems kann eine BET-Oberfläche von mindestens 5 m²/g aufweisen.

Der Anteil an Edelmetall, bezogen auf das Gesamtgewicht aus Edelmetall und Träger, kann 0,01 bis 5 Gew.-%, vorzugsweise 0,05 bis 1,2 Gew.-% und bevorzugt 0,1 bis 0,6 Gew.-% betragen.

Das Edelmetall kann auf oder im Träger verteilt sein.

Der Stoffmengenanteil an Edelmetall, bezogen auf die Stoffmenge der Verbindung E, kann 0,00001 bis 0,1, vorzugsweise 0,0001 bis 0,01, betragen.

Der Stoffmengenanteil an Metalloxid des Katalysatorsystems, bezogen auf die Stoffmenge der Verbindung E, kann 0,01 bis 100, vorzugsweise 0,01 bis 10, betragen.

Das erfindungsgemäße Verfahren kann in organischen Lösemitteln durchgeführt werden, wobei es bevorzugt ist, ohne Lösemittel zu arbeiten und somit keine organischen Lösemittel einzusetzen. Geeignete Lösemittel sind beispielsweise Alkane wie n-Hexan, n-Heptan, n-Tetradecan und Cyclohexan; Ether wie Tetrahydrofuran und Dioxan; Alkanole wie Methanol, Ethanol und t-Butanol; Ester wie Ethylacetat und Butylacetat. Die Lösemittel können für sich allein oder in Mischungen eingesetzt werden. Das Lösemittel wird vorzugsweise in einer Menge eingesetzt, welche das 20-fache oder weniger, bevorzugt das 10-fache oder weniger des Gewichts der Verbindung E, beträgt.

Das erfindungsgemäße Verfahren kann kontinuierlich oder diskontinuierlich ausgeführt werden.

Die Aufreinigung des Ketons kann durch Destillation, Kristallisation oder Umkristallisation erfolgen.

Es ist bevorzugt, dass das erfindungsgemäße Verfahren ohne eine Promoter-Komponente durchgeführt wird. Die Promoterkomponente kann ein Mitglied sein, welches aus den Elementen der Gruppe VIII, der Gruppe Ib, der Gruppe IIb, der Gruppe IIIb, der Gruppe IVb, der Gruppe Vb, der Gruppe VIb und der Gruppe VIIb, Elementen der Lanthaniden sowie Verbindungen der zuvor genannten Elemente ausgewählt wird. Entsprechende Promoter-Komponenten sind in EP-A-1090900 beschrieben. Die zuvor genannten Metalloxide des Katalysatorsystems bzw. alternativen Träger werden nicht als Promoter-Komponente angesehen.

In einer bevorzugten Ausführungsform der Erfindung wird CDAN-Epoxid zu CDON ohne Lösemittel bei Temperaturen von 200 bis 250 °C umgesetzt, wobei als Katalysator ein Palladium-Zirconiumdioxid mit einem Palladium-Anteil von 0,1 bis 0,6 Gew.-%, bezogen auf das Gesamtgewicht des Katalysators, eingesetzt wird. Bei der Reaktion werden maximal 0,9 bar Wasserstoff, ganz besonders bevorzugt maximal 0,5 bar eingesetzt.

Ein weiterer Gegenstand der Erfindung ist ein Katalysatorsystem umfassend Edelmetall und Metalloxid, wobei
I) das Edelmetall nicht geträgert ist; als Metalloxid des Katalysatorsystems ist mindestens Titandioxid oder Zirconiumdioxid enthalten;
II) das Edelmetall geträgert ist und wobei der Träger nicht Titandioxid und/oder Zirconiumdioxid enthält oder daraus besteht; das System enthält zusätzlich zumindest ein Metalloxid, welches ausgewählt ist aus Titandioxid oder Zirconiumdioxid.

Ein weiterer Gegenstand der Erfindung ist die Verwendung mindestens eines Katalysatorsystems zur Katalyse einer Reaktion, bei der mindestens eine Verbindung E zu dem entsprechenden Keton-Derivat umgesetzt wird.

Das erfindungsgemäß zu verwendende Katalysatorsystem umfasst Edelmetall und Metalloxid, wobei
I) das Edelmetall nicht geträgert ist; als Metalloxid des Katalysatorsystems ist mindestens Titandioxid oder Zirconiumdioxid enthalten;
II) das Edelmetall geträgert ist und wobei der Träger nicht Titandioxid und/oder Zirconiumdioxid enthält oder daraus besteht; das System enthält zusätzlich zumindest ein Metalloxid, welches ausgewählt ist aus Titandioxid oder Zirconiumdioxid;
III) das Edelmetall auf einem Metalloxid, welches aus Titandioxid und Zirconiumdioxid ausgewählt ist, geträgert ist.

Im Katalysatorsystem III ist Zirconiumdioxid bevorzugt.

Ein weiterer Gegenstand der Erfindung ist ein Verfahren zur Synthese von Lactamen (erfindungsgemäßes Lactamverfahren), bei dem das zuvor genannte erfindungsgemäße Verfahren zur Herstellung von Ketonen herangezogen wird. Die Verbindung E wird vorzugsweise ausgewählt aus aliphatischen Monoepoxy-Cycloalkane, aliphatischen Monoexpoxy-Cycloalkandienen und aliphatischen Monoepoxy-Cycloalkenen, wobei Monoepoxy-Cycloalkane bevorzugt sind.

Sofern das Keton in einem Gemisch mit dem entsprechenden Alkohol-Derivat vorliegt, kann eine Dehydrierung des Alkohols zum Keton erfolgen. Anschließend kann das Keton oximiert werden. Im Folgeschritt kann die Beckmann-Umlagerung zum Lactam erfolgen, wobei die Umlagerung mittels Schwefelsäure oder Cyanurchlorid erfolgen kann. Die Lactame können unter Polykondensation zu Polyamiden weiterverarbeitet werden.

Die Dehydrierung, die Oximierung, die Beckmann-Umlagerung sowie die Kondensationsreaktion sind dem Fachmann bekannt.

In einer bevorzugten Ausführungsform des erfindungsgsgemäßen Lactamverfahrens wird aus Monoepoxy-cyclododecan (bzw. Cyclododecanepoxid oder 1,2-Cyclododecanepoxid) Laurinlactam hergestellt.

Im Rahmen des bevorzugten Lactamverfahrens kann Cyclododecanepoxid gewonnen werden durch folgende Reaktionsschritte: 1,3-Butadien wird durch Cyclotrimerisation zu Cyclododecatrien umgesetzt. Anschließend erfolgt eine Hydrierung zum Cyclododecen. Durch nachfolgende Epoxidierung wird das Cyclododecanepoxid erhalten. Der Fachmann auf dem Gebiet der Synthese organischer Verbindungen kann andere aliphatische und cycloaliphatische Verbindungen E in Analogie zu der Synthese von Cyclododecanepoxid herstellen.

Nachfolgend wird die vorliegende Erfindung anhand von Beispielen näher erläutert. Alternative Ausführungsformen der vorliegenden Erfindung sind in analoger Weise erhältlich.

### Beispiele

Die prozentuale Angabe bei Katalysatoren gibt den Gewichtsanteil des Edelmetalls an, bezogen auf das Gesamtgewicht des Katalysators umfassend Edelmetall und Träger. Die Abkürzung "calc." steht für "calciniert". Die Abkürzungen der Stoffe sind: CDAN: Cyclododecan; CDEN: Cyclododecen; ECD: Epoxycyclododecan; CDON: Cyclododecanon; CDOL: Cyclododecanol; CDENOL: Cyclododecen-3-ol.

### Beispiel 1: Aluminiumoxidträger (Stand der Technik)

Die Reaktion wurde in einem 500 mL Rundkolben mit mechanischer Rührung durchgeführt. Die Beheizung des Reaktors erfolgte mit einem elektrischen Aluminiumheizblock und die Innentemperatur wurde mit einem Thermofühler kontrolliert. Der Kolben wurde mit 50 mL 1,2-Cyclododecanepoxid und 10 g Katalysator, geträgert auf Aluminiumoxid als Formkörper, gefüllt. Das Katalysatorbett lag am Boden des Kolbens und das flüssige Reaktionsgemisch wurde über dem Bett gerührt. Der Kolben wurde anschließend mit Stickstoff inertisiert und bis 215 °C Innentemperatur erhitzt. Die Temperatur wurde während 5 Stunden gehalten.

**Tabelle 1. Zusammensetzung (Flächen-%, GC) der Reaktionsmischung (5 h)**

| **Katalysator** | **Träger** | **Umsatz (%)** | **Selektivität (%)** | | | | |
|---|---|---|---|---|---|---|---|
| | | | **CDAN** | **CDEN** | **CDON** | **CDOL** | **CDENOL** |
| Ru/Al₂O₃ 1,5 % | γ-*Al₂O₃* | 99 | 0,2 | 5 | 71 | 7 | 1,3 |
| Pd/Al₂O₃ 0,5% | γ-*Al₂O₃* | 100 | 1 | 4 | 75 | 6 | 0 |

### Beispiel 2: Verschiedene Metalle und Träger (erfindungsgemäß)

Die Reaktion wurde in einem 500 mL Rundkolben mit mechanischer Rührung durchgeführt. Die Beheizung des Reaktors erfolgte mit einem elektrischen Aluminiumheizblock und die Innentemperatur wurde mit einem Thermofühler kontrolliert. Der Kolben wurde mit 50 mL 1,2-Cyclododecanepoxid und 10 g Katalysatorsystems III (Edelmetall geträgert auf Titandioxid bzw. Zirconiumdioxid, je als Formkörper) gefüllt. Das Katalysatorbett lag am Boden des Kolbens und das flüssige Reaktionsgemisch wurde über dem Bett gerührt. Der Kolben wurde anschließend mit Stickstoff inertisiert und bis 215 °C Innentemperatur erhitzt. Die Temperatur wurde während 5 Stunden gehalten.

**Tabelle 2. Zusammensetzung (Flächen-%, GC) der Reaktionsmischung (5 h)**

| **Katalysator** | **Träger** | **Umsatz (%)** | **Selektivität (%)** | | | | |
|---|---|---|---|---|---|---|---|
| | | | **CDAN** | **CDEN** | **CDON** | **CDOL** | **CDENOL** |
| Ru/TiO₂ 1,0 % | *Aerolyst®* 7711 | 89 | 0 | 3 | 86 | 4 | 0,6 |
| Pd/TiO₂ 0,5% | *Aerolyst®* 7711 | 93 | 0,4 | 1 | 90 | 3 | 0 |
| Pd/ZrO₂ 0,5% | *aus Zr(OH)₄ 350°C calc.* | 90 | 0 | 0 | 96 | 3 | 0 |
| Pd/ZrO₂ 0,5% | *t-ZrO₂,* 8 % *Y₂O₃* | 94^{(a)} | 0,2 | 0 | 94 | 3 | 0,3 |

| | | | | | | | |
|---|---|---|---|---|---|---|---|
| (a) 30 g Katalysator und T=235 °C | | | | | | | |

### Beispiel 3: Mischung von Katalysatoren (erfindungsgemäß)

Die Reaktion wurde in einem 500 mL Rundkolben mit mechanischer Rührung durchgeführt. Die Beheizung des Reaktors erfolgte mit einem elektrischen Aluminiumheizblock und die Innentemperatur wurde mit einem Thermofühler kontrolliert. Der Kolben wurde mit 50 mL 1,2-Cyclododecanepoxid, 10 g geträgertes Edelmetall (0,5 Gew.-% Palladium auf Siliciumdioxid als Formkörper) und 10 bzw. 20 g Metalloxid des Katalysatorsystems (Formkörper) gefüllt (Katalysatorsystem II). Das Katalysatorbett lag am Boden des Kolbens und das flüssige Reaktionsgemisch wurde über dem Bett gerührt. Der Kolben wurde anschließend mit Stickstoff inertisiert und bis 215 °C Innentemperatur erhitzt. Die Temperatur wurde während 5 Stunden gehalten.

**Tabelle 3. Zusammensetzung (Flächen-%, GC) der Reaktionsmischung (5 h)**

| **Katalysator (10 g)** | **Oxid** | **Umsatz (%)** | **Selektivität (%)** | | | | |
|---|---|---|---|---|---|---|---|
| | | | **CDAN** | **CDEN** | **CDON** | **CDOL** | **CDENOL** |
| | ---* | 7 | 0 | 0 | 97 | 0 | 0 |
| | 10 g TiO₂ *Aerolyst®* 7711 | >99 | 0,3 | 2 | 90 | 2 | 0 |
| Pd/SiO₂ 0,5% | 10 g TiO₂ *Hombikat M234* | >99 | 0,6 | 12 | 77 | 0,7 | 0 |
| | 20 g m-ZrO₂ *m*-*ZrO₂* | 68 | <0,1 | 0 | 98 | 1 | 0 |
| | 20 g ZrO₂ *aus Zr(OH)₄ 350°C calc.* | >99 | 0 | 0 | 95 | 3 | 0 |

| | | | | | | | |
|---|---|---|---|---|---|---|---|
| * nicht erfindungsgemäß | | | | | | | |

### Beispiel 4: Reaktion mit Vollumsatz (erfindungsgemäß)

Die Reaktion wurde in einem 500 mL Rundkolben mit mechanischer Rührung durchgeführt. Die Beheizung des Reaktors erfolgte mit einem elektrischen Aluminiumheizblock und die Innentemperatur wurde mit einem Thermofühler kontrolliert. Der Kolben wurde mit 50 mL 1,2-Cyclododecanepoxid und Katalysator gefüllt (Palladium auf Titandioxid als Formkörper bzw. auf Zirconiumdioxid als Formkörper; Katalysatorsystem III). Das Katalysatorbett lag am Boden des Kolbens und das flüssige Reaktionsgemisch wurde über dem Bett gerührt. Der Kolben wurde anschließend mit Stickstoff inertisiert und bis die gewünschte Innentemperatur erhitzt. Die Temperatur wurde während der gegebenen Zeit gehalten, bis einen vollen Umsatz des Epoxids erreicht wurde.

**Tabelle 4. Zusammensetzung (Flächen-%, GC) der Reaktionsmischung (5 h)**

| **Katalysator** | **Träger** | **Temp. (°C)** | **Zeit (h)** | **Ausbeute (%)** | | | | **CDON +CDOL** |
|---|---|---|---|---|---|---|---|---|
| | | | | **CDAN** | **CDEN** | **CDON** | **CDOL** | |
| 20 g Pd/TiO₂ 0,5% | *Aerolyst®* 7711 | 215 | 5 | 0,7 | 1,7 | 92,1 | 2,1 | **94,2** |
| 10 g Pd/TiO₂ 0,5% | *Aerolyst®* 7711 | 215 | 10* | 1,8 | 0 | 95,7 | 2,5 | **98,2** |
| 30 g Pd/ZrO₂ 0,5% | *t-Zro₂, mit 8 Gew.-% Y₂O₃* | 235 | 10 | 0,2 | 0 | 96,2 | 3,4 | **99,6** |
| 30 g Pd/ZrO₂ 0,5% | *aus Zr(OH)₄ 350°C calc.* | 235 | 5 | 0,5 | 0,5 | 93,5 | 5,5 | **99** |
| 10 g Pd/ZrO₂ 0,5% | *aus Zr(OH)₄ 350°C calc.* | 235 | 10 | 0,3 | 0,1 | 95,3 | 2,9 | **98,2** |

| | | | | | | | | |
|---|---|---|---|---|---|---|---|---|
| * H₂/N₂-Mischung (1 bar mit 90 Vol.-% H₂ und 10 Vol.-% N₂) wurde während der letzten Stunde der Reaktion eingespeist | | | | | | | | |

### Beispiel 5: Reaktion mit verschiedenen Wasserstoffdrücken (erfindungsgemäß)

Die Reaktion wurde in einem 100 mL Autoklav mit Magnetrührer durchgeführt. Die Beheizung des Reaktors erfolgte mit einem 200 °C Ölbad. Der Autoklav wurde mit 20 mL 1,2-Cyclododecanepoxid und 4 g Katalysatorsystem III (Palladium, geträgert auf Titandioxid-Pulver (gemörserter Aerolyst® 7711 Träger)) gefüllt. Er wurde mit Stickstoff inertisiert, bis die gewünschte Innentemperatur erhitzt und anschließend mit Wasserstoff bis zum gewünschten Druck aufgedrückt. Die Öltemperatur wurde während 24 h gehalten.

**Tabelle 5. Zusammensetzung (Flächen-%, GC) der Reaktionsmischung (5 h)**

| **Katalysator (4 g)** | **H₂ Druck (bar)** | **Umsatz (%)** | **Selektivität (%)** | | | | |
|---|---|---|---|---|---|---|---|
| | | | **CDAN** | **CDEN** | **CDON** | **CDOL** | **CDENOL** |
| | 0 | 83 | 0,4 | 0 | 97 | 2 | 0 |
| Pd/TiO₂ 0,5% | 3* | 91 | 0,8 | 0 | 87 | 12 | 0 |
| | 5* | 94 | 1,6 | 0 | 67 | 31 | 0 |
| | 7* | 89 | 1,1 | 0 | 59 | 39 | 0 |

| | | | | | | | |
|---|---|---|---|---|---|---|---|
| * nicht erfindungsgemäß | | | | | | | |

### Beispiel 6: Mischung von Pd-Pulver und TiO₂ (erfindungsgemäß)

Die Reaktion wurde in einem 500 mL Rundkolben mit mechanischer Rührung durchgeführt. Die Beheizung des Reaktors erfolgte mit einem elektrischen Aluminiumheizblock und die Innentemperatur wurde mit einem Thermofühler kontrolliert. Der Kolben wurde mit 50 mL 1,2-Cyclododecanepoxid, 10 g TiO₂ Pulver und Palladium(II)oxid-Pulver als Edemetall (Katalysatorsystem I) gefüllt. Das Katalysatorbett lag am Boden des Kolbens und das flüssige Reaktionsgemisch wurde über dem Bett gerührt. Der Kolben wurde anschließend mit Stickstoff inertisiert und bis 215 °C Innentemperatur erhitzt. Die Temperatur wurde während 5 Stunden gehalten.

**Tabelle 6. Zusammensetzung (Flächen-%, GC) der Reaktionsmischung (5 h)**

| **Metalloxid** | **Edelmetall** | **Umsatz (%)** | **Selektivität (%)** | | | | |
|---|---|---|---|---|---|---|---|
| | | | **CDAN** | **CDEN** | **CDON** | **CDOL** | **CDENOL** |
| ---* | 0,12 g PdO *(Pulver)* | Kein Umsatz | | | | | |
| 10 g TiO₂ *Aerolyst®* 7711 | kein | 90 | 0 | 6 | 3 | 0 | 84 |
| 10 g TiO₂ *Aerolyst®* 7711 | 0,12 g PdO *Pulver* | 79 | 0 | 5 | 46 | 0 | 22 |
| 10 g TiO₂ *Aerolyst®* 7711 | 0,76 g PdO *Pulver* | 91 | 0 | 9 | 68 | 3 | 1 |

| | | | | | | | |
|---|---|---|---|---|---|---|---|
| * nicht erfindungsgemäß | | | | | | | |

### Beispiel 7: Reaktion mit ZrO₂ Pulver (erfindungsgemäß)

Die Reaktion wurde in einem 500 mL Rundkolben mit mechanischer Rührung durchgeführt. Die Beheizung des Reaktors erfolgte mit einem elektrischen Aluminiumheizblock und die Innentemperatur wurde mit einem Thermofühler kontrolliert. Der Kolben wurde mit 50 mL 1,2-Cyclododecanepoxid und dem Katalysatorsystem gefüllt. Das Pd/SiO₂ Katalysatorbett lag - soweit vorhanden - am Boden des Kolbens. Das flüssige Reaktionsgemisch mit ZrO₂-Pulver bzw. Pd geträgert auf ZrO₂ (Pulver) wurde über dem Bett gerührt. Der Kolben wurde anschließend mit Stickstoff inertisiert und bis 215 °C Innentemperatur erhitzt. Die Temperatur wurde während 5 Stunden gehalten.

**Tabelle 7. Zusammensetzung (Flächen-%, GC) der Reaktionsmischung (5 h)**

| **Katalysatorsystem** | **Umsatz (%)** | **Selektivität (%)** | | | | |
|---|---|---|---|---|---|---|
| | | **CDAN** | **CDEN** | **CDON** | **CDOL** | **CDENOL** |
| 10 g Pd/SiO₂ 0,5% *(Extrudat)* + 20 g ZrO₂ (*Pulver, 300°C calciniert*) (System II) | > 99 | 0 | 0 | 95 | 3 | 0 |
| 10 g Pd/SiO₂ 0,5% *(Extrudat)* + 10 g ZrO₂ *(Pulver, 300°C calciniert*) (System II) | 75 | 0 | 0 | 92 | 2 | 0 |
| 10 g Pd/ZrO₂ 0,5 % *(Pulver, 300°C calciniert*) (System III) | 75 | 0 | 0 | 96 | 2 | 0 |

### Beispiel 8: Kontinuierliches Verfahren (erfindungsgemäß)

Die Reaktion wurde in einer kontinuierlich betriebenen Anlage durchgeführt. Die Anlage bestand aus einem Festbettreaktor (ca. 100 mL Katalysatorschüttung) und einem Vorlagestahlbehälter (1 L) mit Standmessung. Der Festbettreaktor wurde mit 100 g Pd/ZrO₂ (0,5 %; System III) und der Behälter mit 950 g CDAN-Epoxid befüllt. Die Flüssigkeit wurde im Kreis aus der Vorlage durch das Katalysatorbett zurück in den Vorlagebehälter mittels einer Umwälzpumpe (21 l/h) gepumpt. Der Reaktor wurde bis zur gewünschten Innentemperatur im Reaktionsgemisch mit einer elektrischen Heizung aufgeheizt. Nachdem ein Umsatz von 80% erreicht wurde, wurde frisches Feed (CDAN-Epoxid) in die Anlage zudosiert. Das Produkt wurde kontinuierlich aus der Anlage ausgetragen, so dass der Füllstand im Vorlagebehälter konstant blieb. Das Reaktionsgemisch wurde mit Stickstoff durch das Festbett und den Behälter geleitet. Die Reaktion wurde ohne Wasserstoff (Tabelle 8, Zeile 1) und mit Wasserstoff (Partialdruck 0,25 bar; Tabelle 8, Zeile 2) durchgeführt.

**Tabelle 8. Zusammensetzung (Flächen-%, GC) der Reaktionsmischung**

| **Druck** | **Temp (°C)** | **Feed (g/h)** | **Umsatz (%)** | **Selektivität (%)** | | | | |
|---|---|---|---|---|---|---|---|---|
| | | | | **CDAN + CDEN** | **CDON** | **CDOL** | **CDEN-ON** | **CDENOL** |
| 1,1 bar N₂ | 215 | 12 | 80,4 | 3,2 | 89 | 1,7 | 3,2 | 2,1 |
| **0 bar H₂** | | | | | | | | |
| 1,4 bar N₂ | 205 | 34 | 84,7 | 9,2 | 84 | 5,1 | 0 | 0,1 |
| **0,25 bar H₂** | | | | | | | | |

## Patentansprüche

1. Verfahren zur Herstellung eines Ketons aus einer mindestens eine Epoxidgruppe enthaltenden Verbindung E unter Einsatz eines Gemisches umfassend mindestens ein Edelmetall und mindestens ein Metalloxid als Katalysatorsystem, **dadurch gekennzeichnet, dass** das Metalloxid des Katalysatorsystems ausgewählt ist aus Titandioxid, Zirconiumdioxid oder Mischungen der beiden Oxide und wobei das Verfahren bei 0 bis 0,9 bar Wasserstoff durchgeführt wird.

2. Verfahren nach Anspruch 1, **dadurch gekennzeichnet, dass** die Verbindung E cycloaliphatisch ist.

3. Verfahren nach einem der vorherigen Ansprüche, **dadurch gekennzeichnet, dass** das Edelmetall ausgewählt ist aus Ruthenium, Palladium und Platin.

4. Verfahren nach einem der vorherigen Ansprüche, **dadurch gekennzeichnet, dass** die Verbindung E vier bis 20 C-Atome umfasst.

5. Verfahren nach einem der vorherigen Ansprüche, **dadurch gekennzeichnet, dass** das Verfahren bei 0 bis 0,5 bar Wasserstoff durchgeführt wird.

6. Verfahren nach einem der vorherigen Ansprüche, **dadurch gekennzeichnet, dass** das Katalysatorsystem ausgewählt ist aus einem der Systeme I, II oder III, wobei
I. das Edelmetall nicht geträgert ist; als Metalloxid des Katalysatorsystems ist mindestens Titandioxid oder Zirconiumdioxid enthalten; oder
II. das Edelmetall geträgert ist und wobei der Träger nicht Titandioxid und/oder Zirconiumdioxid enthält oder daraus besteht; das System enthält zusätzlich zumindest ein Metalloxid, welches ausgewählt ist aus Titandioxid oder Zirconiumdioxid; oder
III. das Edelmetall auf einem Metalloxid geträgert, welches aus Titandioxid und Zirconiumdioxid ausgewählt ist.

7. Verfahren nach einem der vorherigen Ansprüche, **dadurch gekennzeichnet, dass** das Verfahren ohne Wasserstoff durchgeführt wird.

8. Katalysatorsystem umfassend Edelmetall und Metalloxid, wobei
I. das Edelmetall nicht geträgert ist; als Metalloxid des Katalysatorsystems ist mindestens Titandioxid oder Zirconiumdioxid enthalten; oder
II. das Edelmetall geträgert ist und wobei der Träger nicht Titandioxid und/oder Zirconiumdioxid enthält oder daraus besteht; das System enthält zusätzlich zumindest ein Metalloxid, welches ausgewählt ist aus Titandioxid oder Zirconiumdioxid.

9. Verwendung eines Katalysatorsystems umfassend Edelmetall und Metalloxid zur Katalyse einer Reaktion, bei der mindestens eine Epoxidgruppe enthaltende Verbindung E zu dem entsprechenden Keton-Derivat umgesetzt wird, wobei
I. das Edelmetall nicht geträgert ist; als Metalloxid des Katalysatorsystems ist mindestens Titandioxid oder Zirconiumdioxid enthalten; oder
II. das Edelmetall geträgert ist und wobei der Träger nicht Titandioxid und/oder Zirconiumdioxid enthält oder daraus besteht; das System enthält zusätzlich zumindest ein Metalloxid, welches ausgewählt ist aus Titandioxid oder Zirconiumdioxid; oder
III. das Edelmetall auf Zirconiumdioxid als Metalloxid geträgert ist.

10. Verfahren zur Synthese von Lactamen, bei dem ein Verfahren zur Herstellung von Ketonen gemäß einem der Ansprüche 1 bis 7 herangezogen wird.

11. Verfahren nach Anspruch 10, **dadurch gekennzeichnet, dass** als Verbindung E 1,2-Cyclododecanepoxid eingesetzt wird.

## Claims

1. Process for preparing a ketone from a compound E containing at least one epoxy group using a mixture comprising at least one noble metal and at least one metal oxide as a catalyst system, **characterized in that** the metal oxide in the catalyst system is selected from titanium dioxide, zirconium dioxide and mixtures of the two oxides, and wherein the process is conducted at 0 to 0.9 bar of hydrogen.

2. Process according to Claim 1, **characterized in that** the compound E is cycloaliphatic.

3. Process according to any of the preceding claims, **characterized in that** the noble metal is selected from ruthenium, palladium and platinum.

4. Process according to any of the preceding claims, **characterized in that** the compound E comprises four to 20 carbon atoms.

5. Process according to any of the preceding claims, **characterized in that** the process is conducted at 0 to 0.5 bar of hydrogen.

6. Process according to any of the preceding claims, **characterized in that** the catalyst system is selected from one of systems I, II and III, wherein
I. the noble metal is unsupported; the metal oxide present in the catalyst system is at least titanium dioxide or zirconium dioxide; or
II.the noble metal is supported and wherein the support does not comprise or consist of titanium dioxide and/or zirconium dioxide; the system additionally comprises at least one metal oxide selected from titanium dioxide and zirconium dioxide; or
III. the noble metal is supported on a metal oxide selected from titanium dioxide and zirconium dioxide.

7. Process according to any of the preceding claims, **characterized in that** the process is conducted without hydrogen.

8. Catalyst system comprising noble metal and metal oxide, wherein
I. the noble metal is unsupported; the metal oxide present in the catalyst system is at least titanium dioxide or zirconium dioxide; or
II.the noble metal is supported and wherein the support does not comprise or consist of titanium dioxide and/or zirconium dioxide; the system additionally comprises at least one metal oxide selected from titanium dioxide and zirconium dioxide.

9. Use of a catalyst system comprising noble metal and metal oxide for catalysis of a reaction in which compound E containing at least one epoxy group is converted to the corresponding ketone derivative, wherein
I. the noble metal is unsupported; the metal oxide present in the catalyst system is at least titanium dioxide or zirconium dioxide; or
II.the noble metal is supported, and wherein the support does not comprise or consist of titanium dioxide and/or zirconium dioxide; the system additionally comprises at least one metal oxide selected from titanium dioxide and zirconium dioxide; or
III. the noble metal is supported on zirconium dioxide as the metal oxide.

10. Process for synthesizing lactams, in which a process for preparing ketones according to any of Claims 1 to 7 is employed.

11. Process according to Claim 10, **characterized in that** the compound E used is 1,2-cyclododecane epoxide.

## Revendications

1. Procédé pour la préparation d'une cétone à partir d'un composé E contenant au moins un groupe époxyde avec utilisation d'un mélange comprenant au moins un métal noble et au moins un oxyde métallique comme système catalytique, **caractérisé en ce que** l'oxyde métallique du système catalytique est choisi parmi le dioxyde de titane, le dioxyde de zirconium ou des mélanges des deux oxydes et le procédé étant réalisé à 0 à 0,9 bar d'hydrogène.

2. Procédé selon la revendication 1, **caractérisé en ce que** le composé E est cycloaliphatique.

3. Procédé selon l'une quelconque des revendications précédentes, **caractérisé en ce que** le métal noble est choisi parmi le ruthénium, le palladium et le platine.

4. Procédé selon l'une quelconque des revendications précédentes, **caractérisé en ce que** le composé E comprend quatre à 20 atomes de carbone.

5. Procédé selon l'une quelconque des revendications précédentes, **caractérisé en ce que** le procédé est réalisé à 0 jusqu'à 0,5 bar d'hydrogène.

6. Procédé selon l'une quelconque des revendications précédentes, **caractérisé en ce que** le système catalytique est choisi parmi un des systèmes I, II ou III,
I. le métal noble n'étant pas supporté ; au moins du dioxyde de titane ou du dioxyde de zirconium étant contenu comme oxyde métallique du système catalytique ; ou
II. le métal noble étant supporté et le support ne contenant pas de dioxyde de titane et/ou de dioxyde de zirconium ou n'étant pas constitué par celui-ci ; le système contenant en outre au moins un oxyde métallique qui est choisi parmi le dioxyde de titane ou le dioxyde de zirconium ; ou
III. le métal noble étant supporté sur un oxyde métallique, qui est choisi parmi le dioxyde de titane et le dioxyde de zirconium.

7. Procédé selon l'une quelconque des revendications précédentes, **caractérisé en ce que** le procédé est réalisé sans hydrogène.

8. Système catalytique contenant du métal noble et de l'oxyde métallique,
I. le métal noble n'étant pas supporté ; au moins du dioxyde de titane ou du dioxyde de zirconium étant contenu comme oxyde métallique du système catalytique ; ou
II. le métal noble étant supporté et le support ne contenant pas de dioxyde de titane et/ou de dioxyde de zirconium ou n'étant pas constitué par celui-ci ; le système contenant en outre au moins un oxyde métallique qui est choisi parmi le dioxyde de titane ou le dioxyde de zirconium.

9. Utilisation d'un système catalytique comprenant du métal noble et de l'oxyde métallique pour la catalyse d'une réaction, lors de laquelle le composé E contenant au moins un groupe époxyde est transformé en dérivé de cétone correspondant,
I. le métal noble n'étant pas supporté ; au moins du dioxyde de titane ou du dioxyde de zirconium étant contenu comme oxyde métallique du système catalytique ; ou
II. le métal noble étant supporté et le support ne contenant pas de dioxyde de titane et/ou de dioxyde de zirconium ou n'étant pas constitué par celui-ci ; le système contenant en outre au moins un oxyde métallique qui est choisi parmi le dioxyde de titane ou le dioxyde de zirconium ; ou
III. le métal noble étant supporté sur du dioxyde de zirconium comme oxyde métallique.

10. Procédé pour la synthèse de lactames, dans lequel un procédé pour la préparation de cétones selon l'une quelconque des revendications 1 à 7 est utilisé.

11. Procédé selon la revendication 10, **caractérisé en ce qu'**on utilise, comme composé E, de l'époxyde de 1,2-cyclododécane.
